# EUROPÄISCHE PATENTSCHRIFT

(11) **EP 1 740 721 B1**
(45) Veröffentlichungstag und Bekanntmachung des Hinweises auf die Patenterteilung: **27.08.2008**
(21) Anmeldenummer: 05740060.8
(22) Anmeldetag: 26.04.2005
(51) Int. Cl.: C12Q 1/68

(54) **VERFAHREN UND ANORDNUNG ZUR DNA-AMPLIFIKATION MITTELS PCR UNTER EINSATZ VON TROCKENREAGENZIEN**
PCR PROCESS AND ARRANGEMENT FOR DNA AMPLIFICATION USING DRY REAGENTS
PROCEDE ET DISPOSITIF D'AMPLIFICATION D'ADN PAR AMPLIFICATION EN CHAINE PAR POLYMERASE AU MOYEN DE REACTIFS SECS

(30) Priorität: 30.04.2004 DE 102004021822
(43) Veröffentlichungstag der Anmeldung: 10.01.2007
(73) Patentinhaber: SIEMENS AKTIENGESELLSCHAFT, 80333 München (DE)
(72) Erfinder: GUMBRECHT, Walter, 91074 Herzogenaurach (DE); PAULICKA, Peter, 91056 Erlangen (DE); STANZEL, Manfred, 91056 Erlangen (DE)
(86) Internationale Anmeldenummer: PCT/EP2005/051874
(87) Internationale Veröffentlichungsnummer: WO 2005/106023

(56) Entgegenhaltungen:
- EP-A- 0 572 057
- WO-A-96/00301
- WO-A-99/33559
- WO-A-02/072262
- DE-A1- 10 236 460
- US-A- 5 550 044
- US-A- 5 599 660
- US-A- 5 830 644
- US-A- 5 972 386
- US-A1- 2001 012 612
- US-A1- 2002 022 261
- US-A1- 2003 073 110
- US-A1- 2004 018 611
- US-B1- 6 221 584
- US-B1- 6 361 958
- US-B1- 6 403 339
- BLAIR P ET AL: "WAX-EMBEDDED PCR REAGENTS" PCR METHODS & APPLICATIONS, COLD SPRING HARBOR LABORATORY PRESS, US, Bd. 4, Nr. 3, 1. Dezember 1994 (1994-12-01), Seiten 191-194, XP000484537 ISSN: 1054-9803
- KAIJALAINEN S ET AL: "AN ALTERNATIVE HOT START TECHNIQUE FOR PCR IN SMALL VOLUMES USING BEADS OF WAX-EMBEDDED REACTION COMPONENTS DRIED IN TREHALOSE" NUCLEIC ACIDS RESEARCH, OXFORD UNIVERSITY PRESS, SURREY, GB, Bd. 21, Nr. 12, 1993, Seiten 2959-2960, XP002027157 ISSN: 0305-1048
- DALE S J: "Direct microtiter plate sequencing of PCR-amplified M13 clones from plaques using dried reagents." BIOTECHNIQUES. FEB 1992, Bd. 12, Nr. 2, Februar 1992 (1992-02), Seiten 194 , 196-197, XP009052693 ISSN: 0736-6205
- MCMILLAN W A: "RAPID REAL-TIME PCR WITH FULLY INTEGRATED SPECIMEN PREPARATION" PHYTOPATHOLOGY, ST. PAUL, MN, US, Bd. 92, Nr. 6, SUPPL, 27. Juli 2002 (2002-07-27), Seite S110, XP008044514 ISSN: 0031-949X
- RAJA SIVA ET AL: "Technology for automated, rapid, and quantitative PCR or reverse transcription-PCR clinical testing." CLINICAL CHEMISTRY. MAY 2005, Bd. 51, Nr. 5, 3. März 2005 (2005-03-03), Seiten 882-890, XP001207245 ISSN: 0009-9147

## Beschreibung

Die Erfindung bezieht sich auf ein Verfahren zur DNA-Amplifikation durch PCR mit Thermozyklisierung der die DNA enthaltenden Substanzen einschließlich der zugehörigen Reagenzien. Daneben bezieht sich die Erfindung auch auf eine zugehörige Anordnung zur Durchführung des Verfahrens.

Zur Nukleinsäureanalytik z.B. von weißen Blutzellen aus Vollblut zur Beantwortung von humangenomischen Fragestellungen müssen zunächst in einem Probenvorbereitungsschritt die Zellen aufgebrochen und anschließend die dabei freigesetzte DNA isoliert werden. Dabei müssen Blutbestandteile, wie z.B. Hämoglobin, Immunoglobuline und Laktoferrin, die eine nachfolgende PCR inhibieren könnten, entfernt werden.

Im Labor werden diese Arbeitsschritte nach hinlänglich bekanntem Stand der Technik durchgeführt. Insbesondere werden zur Isolierung die DNA an sog. Magnet-Beads gebunden. Über externe Magnetfelder können die Magnet-Beads mit der DNA gezielt befördert und an den vorbestimmten Stellen angereichert werden. Die isolierte DNA kann anschließend von den Beads eluiert werden oder zusammen mit den Beads (als DNA-/Bead-Komplex) für die PCR (Polymerase Chain Reaction) eingesetzt werden.

Gemäß dem Stand der Technik wird die isolierte genomische DNA einer PCR-Reagenz-Lösung (Polymerase, Primer, Nukleotide, Puffer, Hilfsstoffe) zugesetzt und der gesamte Ansatz einer für die PCR geeigneten Thermozyklisierung unterworfen.

Die Realisierung letzteren Verfahrens ist vom Vorhandensein von Laborgeräten wie PCR-Gerät (Thermocycler), sog. Eppendorf-Reaktionsgefäße, Pipettier-Geräten, Kühlbehälter für Reagenzien abhängig und muss von geschultem Personal unter Einhaltung von Sicherheitsvorschriften (Infektionsgefahr, Abfallentsorgung ...) durchgeführt werden. Mehrere volumetrische, genaue Dosierungen (Pipettieren) von Reagenzlösungen müssen durchgeführt werden. Zusätzlich sind diese Arbeitsschritte zeitaufwendig.

Aus der EP 0 572 057 A1 ist eine Zusammensetzung für PCR-Reagenzien bekannt, bei dem die Reagenzien mit einem schmelzbaren Material bedeckt werden, um unerwünschte Reaktionen zu verhindern. Weiterhin ist aus der Veröffentlichung in "Nucleic Acids Research", Vol. 20, No. 7, Page 1717 bis 1723, im Einzelnen beschrieben, wie vor der eigentlichen PCR Fehlreaktionen vermieden werden können. Daneben ist in www.bioexpress.com die so genannte Hot-Start-PCR beschrieben, bei der von einer erhöhten Starttemperatur ausgegangen wird.

Die oben beschriebenen Verfahren sind im Prinzip für eine Laboranalyse geeignet. Aus der US 2002/0022261 A1 wird daneben ein System zur miniaturisierten genetischen Analyse und zugehörige Betriebsverfahren beschrieben, bei denen eine Cartridge mit wenigstens einem Eingang zu einem Kanal verwendet wird. Dabei soll in dem Kanal ein Zellaufschluss für eine anschließende PCR erfolgen. Für die PCR werden diesbezügliche Reagenzien bereitgestellt.

Einrichtungen zur DNA-Analyse sind aus der WO 02/072262 A1, der US 5 550 044 A, der US 5 599 660 A und der US 5 972 386 Abekannt. Weiterhin werden Methoden für die PCR in den Veröffentlichungen PCR Met. Appl. Bd. 4, Nr. 3, Seiten 191 bis 194 und Nucl. Acid Research Res., Bd. 21, S. 2959 bis 2960 beschrieben.

Ausgehend von obigem Stand der Technik ist es Aufgabe der Erfindung, die PCR-Reaktion speziell in einer integrierten miniaturisierten Cartridge zu realisieren und dafür eine geeignete Anordnung zu schaffen.

Die Aufgabe ist bei einem Verfahren der eingangs genannten Art erfindungsgemäß durch die Maßnahmen des Patentanspruches 1 gelöst. Eine zugehörige Anordnung ist im Patentanspruch 9 angegeben. Weiterbildungen des Verfahrens und der Anordnung sind Gegenstand der Unteransprüche.

Die Erfindung geht von der WO 02/0072262 A1 mit der Bezeichnung "Analyseeinrichtung" aus. Dort wird bereits die Verwendung von trockengelagerten, bei Raumtemperatur stabile Reagenzien in Mikrokanälen bzw. Mikrokavitäten einer "Chipkarte" beschrieben, die durch Zuführen von Wasser, kurz vor Verwendung, in Lösung gebracht werden. Dabei wird bei diesem Stand der Technik darauf abgestellt, die Trockenreagenzien vorportioniert bereitzustellen, so dass sich nach Auflösung ein quantitatives Analysemittel ergibt. Demgegenüber geht es bei vorliegender Erfindung um die PCR zwecks nachfolgender Analyse, wofür vorher ein Zellaufschluss mit Isolierung von DNA insbesondere aus einer Vollblut-Probe durchgeführt werden muss.

Mit der Erfindung werden folgende Vorteile im Vergleich zur Labormethode erzielt:
- Es erfolgt eine vollständige Integration aller Stoffe und Verfahren in einer geschlossenen einmal verwendbaren Cartridge;
- die Bevorratung der Reagenzien erfolgt in einer bei Raumtemperatur lagerstabilen Form;
- es sind keine manuellen Arbeitsschritte notwendig, außer Injektion der zu amplifizierenden DNA bzw. der Blutprobe bei Integration von Zellaufschluss und PCR;
- es erfolgt kein direkter Kontakt mit gesundheitsgefährdenden Stoffen, indem Blut, Reagenzien und Reagenzien-Abfall in der Cartridge verbleiben;
- eine kompakte Cartridge-Geometrie erlaubt eine effiziente und schnelle Thermozyklisierung;
- die Cartridge ist kostengünstig herzustellen.

Werden PCR-Reagenzien in getrockneter Form vordosiert, so ist auf eine definierte Dosierung von Flüssigkeit hinsichtlich Volumen und Zusammensetzung zu achten, da die Qualität und Quantität der PCR-Reaktion entscheidend von diesen Parametern abhängt. Das Durchströmen von Flüssigkeit durch einen mit Trockenreagenz beschichteten Kanal ohne gründliche Durchmischung könnte zu einer falschen Konzentration der Reagenzien führen.

Bei der Erfindung ist es in vorteilhafter Weise möglich, bei in biologischen Behältnissen, beispielsweise Zellen, gebundener DNA vor der PCR-Reaktion einen Aufschluss des Behältnisses mit Isolierung der DNA vorzunehmen. Mit einem Zellaufschluss können nunmehr insbesondere Vollblut-Proben direkt verarbeitet werden. Insbesondere dann wenn z.B. Magnet-Bead gebundene DNA aus einem Vollblut-Zellaufschluss für die PCR eingesetzt werden soll ist es erforderlich PCR inhibierende Stoffe aus der Probe zu entfernen. Dies kann besonders vorteilhaft durch Fixierung der Magnet-Beads in der PCR-Kammer durch ein Magnetfeld und Waschen der Beads erreicht werden. Bei Verwendung von Trocken-PCR-Reagenzien müssen Mittel vorhanden sein, die ein Auflösen dieser Reagenzien beim Waschvorgang verhindern. Dies wird erfindungsgemäß durch Einführung einer Schutzschicht wie z.B. Paraffin gelöst.

In eigenerfinderischer Weiterbildung können bei der Erfindung zur PCR-Reaktion Array-Anordnungen eingesetzt werden. Damit wird ermöglicht, gleichzeitig Untersuchungen auf verschiedene DNA-Zielsequenzen vorzunehmen, womit eine erhebliche Zeitersparnis verbunden ist.

Eine erfindungsgemäße Anordnung weist folgende Merkmale auf:
- Es ist mindestens ein Mikrokanal bzw. Mikrokavität vorhanden.
   Das im Mikrokanal bzw. vorzugsweise in Mikrokavität eingebrachte PCR-Reagenz hat folgende Eigenschaften:
- Die trockenbaren Substanzen haben vernachlässigbaren Dampfdruck;
- bei den bei Raumtemperatur lagerstabilen Substanzen bleibt die Eigenschaften eine PCR damit ausführen zu können erhalten;
- das Substanzgemisch haftet an Mikrokanal- bzw. Mikrokavität-Wandungen;
- das Substanzgemisch bildet einen dünnen Film;
- das Substanzgemisch ist mit einem nicht wasserlöslichen Medium, insbesondere einer dünnen Paraffin-Schicht, wasserdicht abgedeckt.
   Speziell bei einer Kombination mit einem Zellaufschluss aus Vollblut ergeben sich für das in die Mikrokavität bzw. vorzugsweise im Mikrokanal eingebrachte Lyse-Reagenz folgende Eigenschaften:
- Es liegen Lyseeigenschaften für weiße Blutzellen und/oder andere Zellen Bakterien Viren vor;
- Es werden ebenfalls trockenbare Substanzen mit vernachlässigbarem Dampfdruck verwendet;
- bei den bei Raumtemperatur stabilen Substanzen bleiben die Zellaufschlusseigenschaften erhalten;
- das Substanzgemisch haftet an Mikrokanal-, bzw. Mikrokavität-Wandungen;
- der "Lysekanal" mündet in der PCR-Kavität.

Weitere Einzelheiten und Vorteile der Erfindung ergeben sich aus der nachfolgenden Beschreibung von Ausführungsbeispielen anhand der Zeichnung in Verbindung mit den Patentansprüchen.

Es zeigen:
- Figur 1: eine Draufsicht auf eine Analyseeinrichtung,
- Figur 2 bis Figur 5: jeweils im Längsschnitt einen Ausschnitt aus Figur 1 längs der Linie II - II zur Verdeutlichung der PCR-Vorbereitung, wobei die einzelnen Teilfiguren 2 bis 5 jeweils unterschiedliche Funktionsschritte darstellen,
- Figur 6: eine Draufsicht auf eine Array-Anordnung zur gleichzeitigen Durchführung einer PCR auf unterschiedliche DNA's und
- Figur 7 bis Figur 9: jeweils einen Längsschnitt durch Figur 6 längs der Linie VII - VII in drei verschiedenen Funktionsschritten.

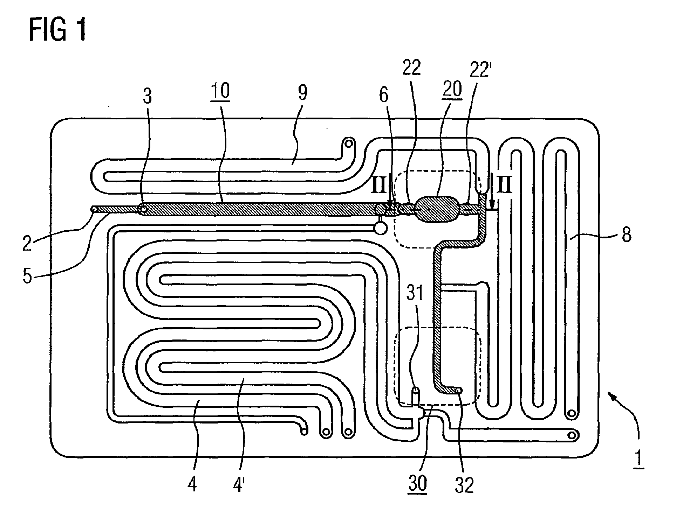

In Figur 1 ist eine Analyseeinrichtung dargestellt, die als zentrale oder dezentrale Messeinrichtung ausgebildet sein kann. Insbesondere ist die Analyseeinrichtung nach Art einer Chipkarte "Lab-on-a-Chip") ausgebildet, die alle Mittel zur Behandlung und Auswertung von Messproben beinhaltet.

Beispielsweise besteht die Einrichtung aus einer Karte 1, welche Ein- und Auslässe aufweist. Insbesondere sind ein Einlass (Port) 2 zum Einbringen von Wasser und ein Einlass 3 (Port) zum Einbringen einer Messprobe, beispielsweise Blut, vorhanden. Dabei ist wesentlich, dass über geeignete Fluidik-Einrichtungen 2 bis 10 Messproben einerseits und Lösungsmittel andererseits zusammengeführt und nach Isolierung der in der Messprobe enthaltenen DNA letztere einer PCR-Kammer 20 zugeführt werden.

Die Fluidik-Einrichtungen beinhalten im Einzelnen neben den bereits erwähnten Wasser- und Probenports 2, 3 zwei Reagenz-Kanäle 4, 4' sowie einen Strömungskanal 5 mit Auslass 6, einen Aufnahmekanal 8 für Abfall und einem weiteren Fluidik-Kanal 9. Mit 10 ist ein zentraler Mischbereich im Strömungskanal 5 für die Probenaufbereitung bezeichnet.

Neben den Mitteln zur PCR enthält die Karte 1 ("Cartridge") weiterhin ein Detektionsmodul 30 mit zugehörigen Anschlüssen für eine Signalverarbeitung. Ferner sind Mittel zur Aufnahme der Rückstände vorhanden. Es wird somit eine Integration gewährleistet, bei dem keine gesundheitsgefährdenden Substanzen nach außen gelangen können.

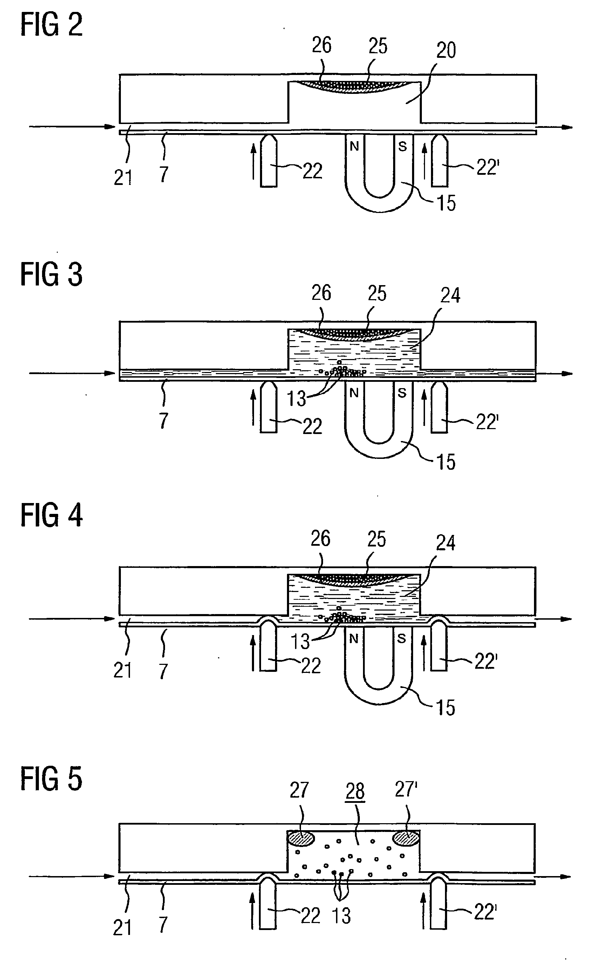

Der Aufschluss einer Vollblutprobe wird im Einzelnen anhand der parallelen Patentanmeldung der Anmelderin mit gleicher Anmeldepriorität mit der Bezeichnung "Verfahren und Anordnung zur DNA-Isolierung mit Trockenreagenzien" beschrieben. Anhand der Figuren 2 bis 5 wird verdeutlicht, wie im Einzelnen die Vorbereitungen für eine DNA-Amplifikation durchgeführt werden:

Es ist eine Kavität 20 vorhanden, in der eine PCR-Reaktion stattfindet. Die PCR(Polymerase Chain Reaction)-Amplifikation ist vom Stand der Technik hinreichend bekannt. Im Einzelnen erfolgt dabei eine Thermozyklierung nach einem vorgegebenen Temperaturprogramm.

In Figur 2 gelangt die Messprobe nach dem Zellaufschluss über einen Strömungskanal 21 in die PCR-Kavität 20. In der PCR-Kavität 20 ist ein lagerstabiles und laufzeitstabiles PCR-Trockenreagenz 25 gelagert. Unter lagerstabil/langzeitstabilem Feststoff ist in diesem Zusammenhang zu verstehen, dass der Feststoff in diesem Zusammenhang mindestens mehrere Monate bei Raumtemperatur gelagert wird unter Erhaltung der die PCR bewirkende Eigenschaft.

Das Trockenreagenz 25 ist wasserlöslich und muss vor der eigentlichen PCR-Reaktion, d.h. im Stadium der Probenaufbereitung und Spülung, vor dem Auflösen und der Denaturierung z.B. durch die Aufschlussreagenzien geschützt werden. Dazu befindet sich auf dem Trockenreagenz 25 ein nicht wasserlösliches Medium 26. Es kann für diesen Zweck insbesondere eine Paraffinschicht verwendet werden.

Das Trennen von zwei Flüssigkeiten mit Hilfe einer erstarrten Paraffin-Trennschicht, mit dem Ziel durch Aufschmelzen des Paraffins die beiden Flüssigkeiten zu vereinigen, ist zwar bereits bekannt. Es wird insbesondere angewandt bei der sog. klassischen "Hot-Start"-PCR. Im vorliegenden Fall wird jedoch keine Flüssigkeit, sondern ein Feststoff mit Paraffin beschichtet.

Weiterhin sind in Figur 2 Ventile 22, 22' und ein Magnet 15 funktionsmäßig angedeutet. Deren Funktion wird nachfolgend bei den weiteren Teilschritten der Figuren 3 bis 5 erläutert:

Aus Figur 3 ist ersichtlich, dass bei geöffneten Ventilen 22, 22' die Messlösung mit den über die Magnet-Beads 12 gebundenen DNA in die Messkavität 20 gelangen. Die DNA konzentriert sich dabei über die Magnetwirkung an den diesbezüglichen Magnetpol. In diesem Stadium kann zunächst ein Spülvorgang erfolgen, wobei nach dem Spülen die Ventile 22, 22' geschlossen werden.

In der entsprechend Figur 4 abgeschlossenen PCR-Kavität 20 befinden sich nunmehr das Reagenz 25, die Abdeckschicht 26 und die wässrige Lösung 24 mit den aufkonzentrierten DNA. Anschließend beginnt die eigentliche PCR-Reaktion. Beim ersten Erwärmen entsprechend der PCR-Thermozyklierung wird gemäß Figur 5 die Paraffinschicht 26 aufgeschmolzen und in kleinen Kügelchen 27, 27' separiert. Das PCR-Reagenz 25 wird dagegen in geeigneter Dosierung im Lösungsmittel bzw. in der Pufferlösung 24 gelöst. In der PCR-Kavität 20 befindet sich dann die aufkonzentrierte, an die Magnet-Bead 13 gebundene DNA als Suspension 28. Nunmehr kann in bekannter Weise die PCR mit mehrfachem Durchlaufen eines Thermozyklisierungsprogrammes durchgeführt werden.

Bei dem in den Figuren 2 bis 5 als PCR-Reagenz vorgesehenen Feststoff geht es darum, eine Substanz mit vernachlässigbarem Dampfdruck auszuwählen, die bei Raumtemperatur lagerstabil ist. Die Substanz kann ein Stoffgemisch sein. Wesentlich ist, dass sie in vordosierter Form langzeitstabil ist. D.h. über mehrere Monate, ihre Eigenschaften die PCR-Reaktion damit ausführen zu können beibehält.

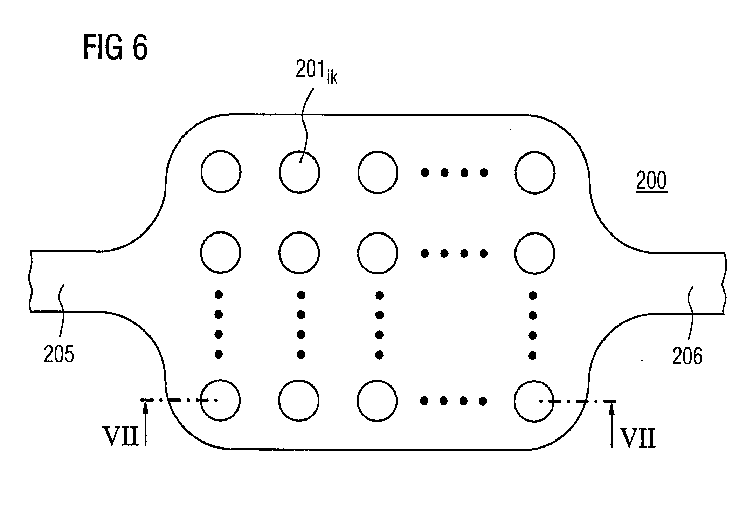

In der Figur 6 ist eine Anordnung zur Durchführung der PCR als Array ausgebildet. Dies bedeutet, dass statt einer einzigen PCR-Kavität, beispielsweise die Kavität 20 in den Figuren 1 bis 5, nunmehr in einem abgeschlossenen Volumen 200 einzelne PCR-Kavitäten 201ᵢₖ vorhanden sind, wobei für die Indices gilt: i = 1 bis m und k = 1 bis n. Die Kammer 200 hat einen Einlass 205 und einen Auslass 206, welcher als Zufluss bzw. Abfluss für Lösungsmittel dienen.

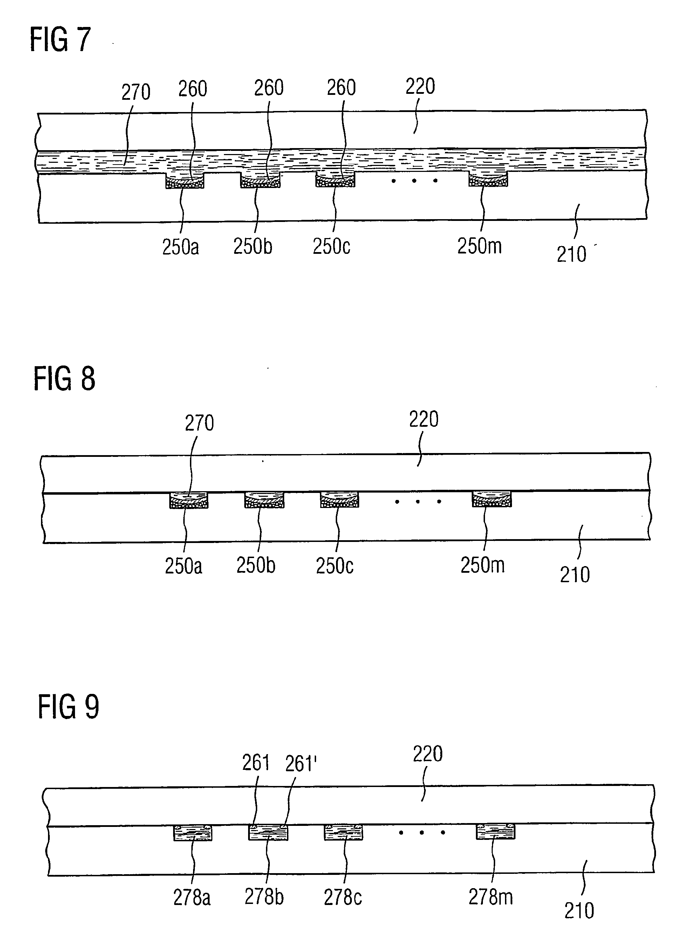

Die Kammer 200 aus Figur 6 besteht aus einem Grundkörper 210 mit als Array verteilten Kavitäten 201ᵢₖ, von dem in Figur 7 eine Zeile n verdeutlicht ist. Es ist weiterhin ein Deckel 220 vorhanden, mit dem die einzelnen PCR-Kavitäten 201ᵢₖ jeweils voneinander getrennt abgeschlossen werden können.

Die einzelnen PCR-Kavitäten 201ᵢₖ werden im Prinzip entsprechend den Figuren 2 bis 5 befüllt. Dies wird aus den Figuren 7 bis 9 deutlich, die als Schnitt eine Reihe einzelner Messkavitäten wiedergibt. Wesentlich ist hier, dass in den Messkavitäten entsprechend einer Zeile ein erster Feststoff 250a, b, c, ... eingebracht ist, wobei dieser Feststoff als Trockenreagenz jeweils spezifisch für eine bestimmte DNA ist. Auf den Feststoffen 250a, b, c, ... befindet sich ein abdichtendes Medium 260. Über den Zufluss 205 wird ein zu untersuchendes DNA-Gemisch 270 in Lösung oder als Suspension zugeführt.

In der Figur 8 ist gezeigt, dass nach dem Zuführen des Lösungsmittels zu den einzelnen Messkavitäten 201ᵢₖ der Deckel 220 geschlossen wird, so dass nunmehr die einzelnen PCR-Kavitäten separiert sind. Wenn nunmehr eine Thermozyklisierung bei geschlossenem Deckel gem. Fig. 9 durchgeführt wird, befinden sich in den einzelnen PCR-Kavitäten jeweils unterschiedliche, für die Amplifikation einer Ziel-DNA spezifische Reagenzien zusammen mit der Proben-DNA. Es können somit gleichzeitig und parallel mehrere PCR-Reaktionen für eine Vielzahl von zu analysierender DNA durchgeführt werden.

Ein PCR-Array entsprechend der Figur 6 kann in eine Analyseneinheit entsprechend Figur 1 integriert werden. Jede PCR-Kavität kann zusätzlich mit einer Sensor-Einrichtung zur Detektion des PCR-Produkts, z.B. mit einer Edelmetallelektrode zur elektrischen Detektion ausgestattet sein. Die Elektroden können außerdem mit Hybridisierungs-Sonden oder auch mit PCR-Primern ausgestattet sein. Es ergeben sich somit verbesserte Anwendungsmöglichkeiten, wie z.B. die Analyse von komplexen DNA-Gemischen, die mit Multiplex-PCR in einem einzigen PCR-Gefäß nicht realisierbar sind.

Bei dem vorstehend beschriebenen Verfahren und der zugehörigen Anordnungen sind folgende Maßnahmen bzw. Merkmale wesentlich:
- im Mikrokanal bzw. Mikrokavität sind Substrate mit DNAbindenden Eigenschaften eingebracht, z.B. DNA-bindende Magnet-Beads;
- die Lyse-Reagenzien und Magnet-Beads können zusammen in einer einzigen Matrix enthalten sein;
- es ist Eingabe-Port für Vollblut-Probe vorhanden;
- es sind Mittel zur Zufuhr von Wasser, z.B. ein Zufluss-Port, zum Anschluss an externe Wasserzufuhr vorhanden;
- im Mikrokanal bzw. Mikrokavität mit den trockenen Puffersubstanzen herrscht nach Wasserzufuhr definierte Ionenstärke;
- es sind Mittel zum Mischen von Vollblutprobe und Wasser bzw. Puffer-Lösung vorhanden;
- es sind Mittel zum Durchströmen des mit Lyse/Bead-Reagenz beschichteten Mikrokanals, bzw. Mikrokavität mit Blut, bzw. Blut/Wasser-, Blut/Puffer-Gemisches vorhanden;
- es sind Mittel zum Generieren eines Magnetfeldes zum Fixieren des DNA/Magnet-Bead-Komplexes in der PCR-Kavität vorhanden;
- es sind Mittel zum Verschließen der PCR-Kavität vorhanden;
- für die PCR sind Mittel zur Thermozyklisierung vorhanden;
- die PCR-Kavität hat Mittel zum mindestens partiellen Druckausgleich.

Wesentliche Vorteile der Anordnung und des Verfahrens sind:
- es erfolgt bereits bei Herstellung der Cartridges eine präzise Dosierung des PCR-Reagenz;
- im Betrieb wird eine präzise Menge und Zusammensetzung des PCR-Reagenz beim Befüllen, bzw. Durchströmen der PCR-Kavität erhalten. Somit liegen keine Veränderungen der Reagenzien-Soll-Konzentration insbesondere Verdünnungs-Effekte vor;
- das Volumen der PCR-Kavität ist beim Befüllen konstant und bekannt;
- nach Aufschmelzen der Paraffin-Trennschicht wird trockenes PCR-Reagenz mit dem Lösungsmittel, insbes. Wasser oder Wasser mit niedrigem Salzgehalt, vereinigt und es kann durch Konvektion eine definierte PCR-Reagenzlösung entstehen;
- bei Kombination mit der Bindung von DNA an Beads (Bead-Technik) muss die DNA nicht extra außerhalb der PCR-Kavität eluiert werden.

Somit ist gewährleistet, dass der gesamte Analysevorgang einschließlich der Probenbereitstellung im abgeschlossenen System, das eine Einmal-Cartridge bildet, erfolgt.

## Patentansprüche

1. Verfahren zur DNA-Amplifikation durch PCR mit Thermozyklisierung der die DNA enthaltenden Substanzen einschließlich der zugehörigen Reagenzien, mit folgenden Maßnahmen:
- Es erfolgt eine vollständige Integration aller Stoffe und Verfahrensschritte in einer geschlossenen Analyseneinheit mit einer einmal verwendbaren Cartridge mit Mikrokanälen oder Mikrokavitäten, in denen zumindest die für die PCR erforderlichen Reagenzien in einer bei Raumtemperatur lagerstabilen Form an wandungen haftend bevorratet sind, wobei
- zunächst die wasserlöslichen Reagenzien durch eine schicht eine nichtwasserlöslichen Mediums abgedeckt werden und
- die zu amplifizierende DNA in einem wässrigen Lösungsmittel zugeführt wird,
- anschließend wird die abdeckende Wirkung des nicht wasserlöslichen Mediums aufgehoben, wodurch
- die wasserlöslichen Reagenzien im wässrigen Lösungsmittel gelöst werden,
- wonach die PCR-Thermozyklisierungsreaktionen gestartet werden.

2. Verfahren nach Anspruch 1, **dadurch gekennzeichnet, dass** als Reagenzien für die PCR wasserfreie, über mehrere Monate bei Raumtemperatur lagerbare Substanzen verwendet werden, die nach Wasserzufuhr die Durchführung einer PCR ermöglichen.

3. Verfahren nach Anspruch 1, **dadurch gekennzeichnet, dass** bei in biologischen Behältnissen gebundener DNA, beispielsweise einer Vollblutprobe, eine Integration des Aufschlusses der Behältnisse, beispielsweise des Zellaufschlusses von weißen Blutzellen, mit der PCR erfolgt.

4. Verfahren nach Anspruch 1, **dadurch gekennzeichnet, dass** eine Analyseneinheit (Cartridge) mit solcher Geometrie verwendet wird, die eine effiziente und schnelle Thermozyklisierung ermöglicht.

5. Verfahren nach einem der vorhergehenden Ansprüche, **dadurch gekennzeichnet, dass** als Analyseneinheit zur Ausführung der Reaktionen ein Einmalprodukt verwendet wird, das in Massenfertigung klein und kostengünstig herzustellen ist.

6. Verfahren nach einem der vorhergehenden Ansprüche, **dadurch gekennzeichnet, dass** die PCR-Reaktionen in einer Array-Anordnung mit gegebenenfalls verschiedenen Primern parallel für unterschiedliche DNA-Amplifikationen durchgeführt wird.

7. Verfahren nach Anspruch 6, **dadurch gekennzeichnet, dass** für die unterschiedlichen DNA-Amplifikationen unterschiedliche zusammengesetzte PCR-Reagenzien verwendet werden.

8. Verfahren nach Anspruch 6, **dadurch gekennzeichnet, dass** das Array mit einer DNA-Probe befüllt wird, der Deckel (210) die PCR-Kavitäten (250ᵢₖ) abschließt und voneinander fluidisch isoliert, die Paraffinschichten (260) aufgeschmolzen werden und individuelle DNA-Amplifikationen parallel durchgeführt werden.

9. Anordnung zur Durchführung des Verfahrens nach Anspruch 1 oder einem der Ansprüche 2 bis 8, mit einer Analyseneinheit, welche als einmal verwendbare Cartridge ausgeführt ist, wobei wenigstens ein Mikrokanal (5) und/oder Mikrokavität (20) zur Aufnahme eines PCR-Reagens vorhanden ist worin die PCR-Reagenzien als Gemisch, das bei Raumtemperatur eine stabile Substanz bildet, eingebracht sind , und mit einer Schicht (26) eines wasserunlöslichen Mediums abgedeckt sind, wobei der Feststoff (25) an den Wandungen des Mikrokanals (5) bzw. der Mikrokavität (20) haftet.

10. Anordnung nach Anspruch 9, **dadurch gekennzeichnet, dass** das Gemisch ein wasserfreier Feststoff ist.

11. Anordnung nach Anspruch 9, **dadurch gekennzeichnet, dass** mittels eines Filmbildners ein PCR-Reagenz-Film gebildet ist.

12. Anordnung nach Anspruch 9, **dadurch gekennzeichnet, dass** das wasserunlösliche Medium Paraffin ist.

13. Anordnung nach Anspruch 9, **dadurch gekennzeichnet, dass** das Paraffin zur Freigabe der PCR-Reagenzien einen definierter Schmelzpunkt hat.

14. Anordnung nach einem der Ansprüche 9 bis 13, **dadurch gekennzeichnet, dass** zur Durchführung eines Zellaufschlusses in der Mikrokavität (20) bzw. im Mikrokanal (5) weiterhin ein Lyse-Reagenz vorhanden ist, welches spezifische Eigenschaften z.B. für weiße Blutzellen.

15. Anordnung nach Anspruch 14, **dadurch gekennzeichnet, dass** das Lyse-Reagenz aus trockenbaren Substanzen mit vernachlässigbarem Dampfdruck besteht, die bei Raumtemperatur lagerstabil sind und das Lyse-Reagenz an den Wandungen des der PCR-Kavität (20) vorgeschalteten Mikrokanals (5) haftet, wobei der Kanal (5) für die Lyse-Substanz in die PCR-Kavität (20) mündet.

16. Anordnung nach einem der Ansprüche 9 bis 15, **dadurch gekennzeichnet, dass** im Mikrokanal (5) bzw. in der Mikrokavität (20) Substrate vorhanden sind, die DNA bindende Eigenschaften haben.

17. Anordnung nach Anspruch 16, **dadurch gekennzeichnet, dass** die Substrate DNA-bindende so genannte Magnet-Beads sind.

18. Anordnung nach einem der Ansprüche 9 bis 17, **dadurch gekennzeichnet, dass** die Lyse-Reagenzien und die Magnet-Beads in einer gemeinsamen Trockenmatrix enthalten sind.

19. Anordnung nach einem der Ansprüche 9 bis 18, **dadurch gekennzeichnet, dass** ein Eingabeport (2) für Vollblut-Proben vorhanden ist.

20. Anordnung nach einem der Ansprüche 9 bis 19, **dadurch gekennzeichnet, dass** Mittel zur Zufuhr von Wasser vorhanden sind.

21. Anordnung nach einem der Ansprüche 9 bis 20, **dadurch gekennzeichnet, dass** Mittel zum Mischen von Blutblutprobe und Wasser, bzw. einer Pufferlösung, vorhanden sind.

22. Anordnung nach einem der Ansprüche 9 bis 21, **dadurch gekennzeichnet, dass** Mittel zum Durchströmen des mit Lyse/Bead-Reagenz beschichteten Mikrokanals (5) bzw. Mikrokavität (20) mit Blut, Blut/Wasser- oder Blut/Puffer-Gemische vorhanden sind.

23. Anordnung nach einem der Ansprüche 9 bis 22, **dadurch gekennzeichnet, dass** Mittel (25) zum Generieren eines Magnetfeldes zwecks Fixieren eines DNA-Magnet-Bead-Komplexes in der PCR-Kavität (20) vorhanden sind.

24. Anordnung nach einem der Ansprüche 9 bis 23, **dadurch gekennzeichnet, dass** Mittel zur Thermozyklisierung vorhanden sind.

25. Anordnung nach einem der Ansprüche 9 bis 24, **dadurch gekennzeichnet, dass** Mittel zum zumindest eines partiellen Druckausgleiches in der PCR-Kavität vorhanden sind.

26. Anordnung nach einem der Ansprüche 9 bis 25, **dadurch gekennzeichnet, dass** ein Array von PCR-Kavitäten (210ᵢₖ) vorhanden ist.

27. Anordnung nach einem der Ansprüche 9 bis 26, **dadurch gekennzeichnet, dass** in den PCR-Kavitäten (250ᵢₖ) unterschiedliche PCR-Reagenzien (251a,b, ...) bevorratet sind.

28. Anordnung nach einem der Ansprüche 9 bis 27, **dadurch gekennzeichnet, dass** ein Deckel (210) zum Abschluss der PCR-Kavitäten (250ᵢₖ) während der PCR vorhanden ist

## Claims

1. Process for DNA amplification by PCR with thermocycling of the substances containing the DNA together with the associated reagents, having the following measures:
- all the materials and process steps are fully integrated in a closed analysis unit having a single-use cartridge with microchannels or microcavities, in which at least the reagents necessary for the PCR are held in a manner adhering to walls in a form which is stable when stored at room temperature,
- the water-soluble reagents are firstly covered with a layer of a water-insoluble medium and
- the DNA to be amplified is supplied in an aqueous solvent,
- the covering effect of the water-insoluble medium is subsequently negated, so that
- the water-soluble reagents dissolve in the aqueous solvent,
- after which the PCR thermocycling reactions can start.

2. Process according to Claim 1, **characterized in that** water-free substances which can be stored for several months at room temperature, and which make it possible to carry out a PCR after supplying water, are used as reagents for the PCR.

3. Process according to Claim 1, **characterized in that** for DNA bound in biological structures, for example a whole blood sample, the disintegration of the structures, for example the cell disintegration of white blood cells, is integrated with the PCR.

4. Process according to Claim 1, **characterized in that** an analysis unit (cartridge) having a geometry that permits efficient and rapid thermocycling is used.

5. Process according to one of the preceding Claims, **characterized in that** a disposable product that is small and inexpensive to make in mass production is used as the analysis unit for performing the reactions.

6. Process according to one of the preceding Claims, **characterized in that** the PCR reactions are carried out in parallel for different DNA amplifications in an array arrangement, optionally with different primers.

7. Process according to Claim 6, **characterized in that** PCR reagents with different compositions are used for the different DNA amplifications.

8. Process according to Claim 6, **characterized in that** the array is filled with a DNA sample, the lid (210) closes the PCR cavities (250ᵢₖ) and isolates them fluidically from one another, the paraffin layers (260) are melted and individual DNA amplifications are carried out in parallel.

9. Arrangement for carrying out the process according to Claim 1 or one of Claims 2 to 8, having an analysis unit which is designed as a single-use cartridge, at least one microchannel (5) and/or microcavity (20) being provided in order to receive a PCR reagent, wherein the PCR reagents are introduced as a mixture forming a substance that is stable at room temperature and are covered with a layer (26) of a water-insoluble medium, the solid (25) adhering to the walls of the microchannel (5) or of the microcavity (20).

10. Arrangement according to Claim 9, **characterized in that** the mixture is a water-free solid.

11. Arrangement according to Claim 9, **characterized in that** a PCR reagent film is formed by means of a film-forming agent.

12. Arrangement according to Claim 9, **characterized in that** the water-insoluble medium is paraffin.

13. Arrangement according to Claim 9, **characterized in that** the paraffin has a defined melting point for releasing the PCR reagents.

14. Arrangement according to one of Claims 9 to 13, **characterized in that** a lysis reagent, which has specific properties e.g. for white blood cells, is furthermore provided in order to disintegrate cells in the microcavity (20) or in the microchannel (5).

15. Arrangement according to Claim 14, **characterized in that** the lysis reagent consists of dryable substances with a negligible vapor pressure, which are stable when stored at room temperature, and the lysis reagent adheres to the walls of the microchannel (5) upstream of the PCR cavity (20), the channel (5) for the lysis substance opening into the PCR cavity (20).

16. Arrangement according to one of Claims 9 to 15, **characterized in that** substrates which have DNA-binding properties are provided in the microchannel (5) or in the microcavity (20).

17. Arrangement according to Claim 16, **characterized in that** the substrates are DNA-binding, so-called magnet beads.

18. Arrangement according to one of Claims 9 to 17, **characterized in that** the lysis reagents and the magnet beads are contained in a common dry matrix.

19. Arrangement according to one of Claims 9 to 18, **characterized in that** an input port (2) for whole blood samples is provided.

20. Arrangement according to one of Claims 9 to 19, **characterized in that** means for supplying water are provided.

21. Arrangement according to one of Claims 9 to 20, **characterized in that** means are provided for mixing a blood sample and water, or a buffer solution.

22. Arrangement according to one of Claims 9 to 21, **characterized in that** means are provided so that blood, blood/water or blood/buffer mixtures can flow through the microchannel (5) or microcavity (20) coated with lysis/bead reagent.

23. Arrangement according to one of Claims 9 to 22, **characterized in that** means (25) are provided for generating a magnetic field for the purpose of fixing a DNA-magnet bead complex in the PCR cavity (20).

24. Arrangement according to one of Claims 9 to 23, **characterized in that** thermocycling means are provided.

25. Arrangement according to one of Claims 9 to 24, **characterized in that** means are provided for at least partial pressure equilibration in the PCR cavity.

26. Arrangement according to one of Claims 9 to 25, **characterized in that** an array of PCR cavities (210ᵢₖ) is provided.

27. Arrangement according to one of Claims 9 to 26, **characterized in that** different PCR reagents (251a, b, ...) are provided in the PCR cavities (250ᵢₖ).

28. Arrangement according to one of Claims 9 to 27, **characterized in that** a lid (210) is provided for closing the PCR cavities (250ᵢₖ) during the PCR.

## Revendications

1. Procédé d'amplification de l'ADN par réaction en chaîne par polymérase ( PCR ) par thermocyclisation des substances contenant l'ADN, y compris les réactifs associés, le procédé comprenant les étapes suivantes :
- on effectue une intégration complète de toutes les substances et toutes les étapes opératoires dans une unité d'analyse fermée, qui comporte une cartouche à usage unique, dotée de microcanaux ou de microcavités dans lesquels au moins les réactifs nécessaires à la PCR sont stockées sous une forme stable à la température ambiante en adhérant aux parois,
- les réactifs hydrosolubles sont tout d'abord recouverts d'une couche de milieu non hydrosoluble et
- l'ADN à amplifier est amené dans un solvant aqueux,
- l'action de recouvrement du milieu non hydrosoluble est ensuite supprimée de sorte que
- les réactifs hydrosolubles sont dissous dans le solvant aqueux,
- ce qui entraîne le démarrage des réactions de thermocyclisation.

2. Procédé selon la revendication 1, **caractérisé en ce que** l'on utilise comme réactifs pour la PCR des substances anhydres, stockables plusieurs mois à la température ambiante, qui permettent de réaliser la PCR après apport d'eau.

3. Procédé selon la revendication 1, **caractérisé en ce que,** dans le cas d'ADN lié dans des enveloppes biologiques, par exemple un échantillon de sang complet, on intègre la désagrégation des enveloppes, par exemple la cytolyse de cellules leucocytaires, à la PCR.

4. Procédé selon la revendication 1, **caractérisé en ce que** l'on utilise une unité d'analyse ( cartouche ) dont la géométrie permet d'effectuer une thermocyclisation efficace et rapide.

5. Procédé selon l'une des revendications précédentes, **caractérisé en ce que** l'on utilise comme unité d'analyse, pour réaliser les réactions, un produit à usage unique qui peut être fabriqué en série dans de petites dimensions et d'une manière peu coûteuse.

6. Procédé selon l'une des revendications précédentes, **caractérisé en ce que** les réactions PCR sont réalisées dans un agencement en réseau avec des amorces éventuellement différentes en parallèle pour différentes amplification d'ADN.

7. Procédé selon la revendication 6, **caractérisé en ce que** l'on utilise pour les différentes amplifications d'ADN des réactifs de PCR de composition différente.

8. Procédé selon la revendication 6, **caractérisé en ce que** le réseau est rempli d'un échantillon d'ADN, le couvercle ( 210 ) ferme les cavités de PCR ( 250ᵢₖ ) et les isole fluidiquement les unes des autres, les couches de paraffine ( 260 ) sont fondues et les amplifications d'ADN individuelles sont réalisées en parallèle.

9. Dispositif pour mettre en oeuvre le procédé de la revendication 1 ou de l'une des revendications 2 à 8, lequel dispositif comporte une unité d'analyse qui est réalisée sous la forme d'une cartouche à usage unique, au moins un microcanal ( 5 ) et/ou une microcavité ( 20 ) de réception d'un réactif de PCR et les réactifs de PCR étant introduits dans ledit microcanal et/ou ladite microcavité sous la forme d'un mélange qui forme une substance stable à la température ambiante, et étant recouverts d'une couche ( 26 ) d'un milieu non hydrosoluble, la substance solide ( 25 ) adhérant aux parois du microcanal ( 5 ) respectivement de la microcavité ( 20 ).

10. Dispositif selon la revendication 9, **caractérisé en ce que** le mélange est une substance solide anhydre.

11. Dispositif selon la revendication 9, **caractérisé en ce qu'**un film de réactif de PCR est formé au moyen d'un agent filmogène.

12. Dispositif selon la revendication 9, **caractérisé en ce que** le milieu non hydrosoluble est de la paraffine.

13. Dispositif selon la revendication 9, **caractérisé en ce que** la paraffine a un point de fusion défini pour libérer les réactifs de PCR.

14. Dispositif selon l'une des revendications 9 à 13, **caractérisé en ce que,** pour réaliser la cytolyse dans la microcavité ( 20 ) ou le microcanal ( 5 ), il est en outre prévu un réactif de lyse présentant des propriétés spécifiques, par exemple pour des globules sanguins.

15. Dispositif selon la revendication 14, **caractérisé en ce que** le réactif de lyse est constitué de substances pouvant être séchées ayant une pression de vapeur négligeable, et qui sont stables à la température ambiante, et **en ce que** le réactif de lyse adhère aux parois du microcanal ( 5 ) placé en amont de la cavité de PCR ( 20 ), le canal ( 5 ) destiné à la substance de lyse débouchant dans la cavité de PCR ( 20 ).

16. Dispositif selon l'une des revendications 9 à 15, **caractérisé en ce que** des substrats, qui présentent des propriétés de formation d'ADN, sont présents dans le microcanal ( 5 ) respectivement dans la microcavité ( 20 ).

17. Dispositif selon la revendication 16, **caractérisé en ce que** les substrats de formation d'ADN sont ce que l'on appelle des perles magnétiques ( Magnet-beads ).

18. Dispositif selon l'une des revendications 9 à 17, **caractérisé en ce que** les réactifs de lyse et les perles magnétiques sont contenus dans une matrice sèche commune.

19. Dispositif selon l'une des revendications 9 à 18, **caractérisé en ce qu'**il est prévu un orifice d'entrée ( 2 ) d' échantillons de sang complet.

20. Dispositif selon l'une des revendications 9 à 19, **caractérisé en ce qu'**il est prévu des moyens d'apport d'eau.

21. Dispositif selon l'une des revendications 9 à 20, **caractérisé en ce qu'**il est prévu des moyens de mélange d'un échantillon de sang et d'eau, ou d'une solution tampon.

22. Dispositif selon l'une des revendications 9 à 21, **caractérisé en ce qu'**il est prévu des moyens permettant de faire circuler du sang, un mélange sang/eau ou un mélange sang/tampon dans le microcanal ( 5 ) respectivement la microcavité ( 2 ) doté d'une couche de réactif de lyse/perle.

23. Dispositif selon l'une des revendications 9 à 22, **caractérisé en ce qu'**il est prévu des moyens ( 25 ) permettant de produire un champ magnétique permettant de fixer un complexe ADN/perle magnétique dans la cavité de PCR ( 20 ).

24. Dispositif selon l'une des revendications 9 à 23, **caractérisé en ce qu'**il est prévu des moyens de thermocyclisation.

25. Dispositif selon l'une des revendications 9 à 24, **caractérisé en ce qu'**il est prévu des moyens permettant de compenser au moins partiellement la pression dans la cavité de PCR.

26. Dispositif selon l'une des revendications 9 à 25, **caractérisé en ce qu'**il est prévu un réseau de cavités de PCR ( 210ᵢₖ ).

27. Dispositif selon l'une des revendications 9 à 26, **caractérisé en ce que** différents réactifs de PCR ( 251a,b, ... ) sont stockés dans les cavités de PCR ( 250ᵢₖ ).

28. Dispositif selon l'une des revendications 9 à 27, **caractérisé en ce qu'**il est prévu un couvercle ( 210 ) destiné à fermer les cavités de PCR ( 210ᵢₖ ) pendant la PCR.
